# EUROPEAN PATENT SPECIFICATION

(11) **EP 3 812 771 B1**
(45) Date of publication and mention of the grant of the patent: **27.12.2023**
(21) Application number: 19823488.2
(22) Date of filing: 29.05.2019
(51) Int. Cl.: G01N 33/68, G01N 33/577

(54) **METHOD FOR FULL COURSE DETECTION OF C-REACTIVE PROTEIN AND CORRESPONDING KIT**
VERFAHREN ZUM VOLLKURSNACHWEIS VON C-REAKTIVEM PROTEIN UND ZUGEHÖRIGES KIT
PROCÉDÉ DE DÉTECTION DE L'ÉVOLUTION COMPLÈTE DE LA PROTÉINE C RÉACTIVE ET KIT CORRESPONDANT

(30) Priority: 20.06.2018 CN 201810634120
(43) Date of publication of application: 28.04.2021
(73) Proprietor: Xiamen Innodx Biotech Co., Ltd, Xiamen, Fujian 361022 (CN); Xiamen University, Xiamen, Fujian 361005 (CN)
(72) Inventor: CHEN, Zimin, Xiamen, Fujian 361022 (CN); XIONG, Junhui, Xiamen, Fujian 361022 (CN); XU, Weiling, Xiamen, Fujian 361022 (CN); WENG, Zuxing, Xiamen, Fujian 361022 (CN); WANG, Long, Xiamen, Fujian 361022 (CN); ZHENG, Xiaohong, Xiamen, Fujian 361022 (CN); SUN, Xudong, Xiamen, Fujian 361022 (CN); GE, Shengxiang, Xiamen, Fujian 361005 (CN)
(74) Representative: Lavoix
(86) International application number: PCT/CN2019/089015
(87) International publication number: WO 2019/242474

(56) References cited:
- EP-A1- 1 396 724
- EP-A1- 1 679 516
- CN-A- 103 940 992
- CN-A- 103 941 017
- CN-A- 103 941 017
- CN-A- 105 988 003
- CN-A- 105 988 003
- CN-A- 107 632 163
- CN-A- 107 632 163
- CN-A- 107 656 043
- US-A- 5 358 852
- VASHIST SANDEEP KUMAR ET AL: "One-step kinetics-based immunoassay for the highly sensitive detection of C-reactive protein in less than 30min", ANALYTICAL BIOCHEMISTRY, ACADEMIC PRESS, AMSTERDAM, NL, vol. 456, 13 April 2014 (2014-04-13), pages 32-37, XP029026976, ISSN: 0003-2697, DOI: 10.1016/J.AB.2014.04.004 & Anonymous: "A rapid one-step kinetics-based immunoassay procedure for the highly-sensitive detection of C-reactive protein | Zenodo", , 31 December 2014 (2014-12-31), XP055893847, Retrieved from the Internet: URL:https://zenodo.org/record/13656#.YhN5j ejMJPY [retrieved on 2022-02-21]
- TANNE DAVID ET AL: "C-Reactive Protein as a Predictor of Incident Ischemic Stroke Among Patients With Preexisting Cardiovascular Disease", STROKE, vol. 37, no. 7, 1 July 2006 (2006-07-01), pages 1720-1724, XP055893863, US ISSN: 0039-2499, DOI: 10.1161/01.STR.0000227004.08182.bf

## Description

### Technical Field

The invention belongs to the technical field of chemiluminescence immunoassays, and specifically relates to a method for full-range detection of C-reactive protein and a corresponding kit.

### Background

C-Reactive protein (CRP) is an acute phase reaction protein discovered by Tillet and Francis in 1930 that can react with *Streptococcus pneumoniae* C polysaccharide in the presence of Ca²⁺ to form a complex; serum CRP is synthesized by hepatocytes under the stimulation of IL-6, IL-2 and TNF, and inflammatory local macrophages are also produced in small amounts. CRP has a molecular weight of about 115KD and consists of five identical unglycosylated polypeptide subunits, each subunit contains 204 amino acids, these subunits are connected by non-covalent bonds to form a cyclic pentamer, and with an interchain disulfide bond, this pentameric protein has remarkable heat resistance and protein degradation resistance.

CRP is widely distributed in the body. In addition to blood, it can be detected in pleural fluid, ascites, pericardial fluid, and joint fluid.

CRP is an important acute reaction protein. It starts to increase at 6-8h after the occurrence of bacterial infection and reaches a peak at 24-48h. After the infection is eliminated, its content drops sharply and returns to normal within a week.

The clinical application of CRP is mainly used as a first-choice indicator to identify bacterial or viral infections, as well as to monitor disease changes and postoperative infections, to dynamically observe the efficacy of antibiotics, to guide and monitor treatments and the like. CRP is also related to cardiovascular disease, coronary heart disease, and acute coronary syndrome, in which the level of CRP in patients is often significantly elevated, and the degree of the elevated level is significantly correlated to the degree of coronary artery obstruction, the occurrence and prognosis of the end-event of coronary heart disease, and congestive heart failure. In addition, CRP is also an independent predictor of atrial fibrillation, and there is a certain correlation between serum CRP concentration and hypertension. The systolic and diastolic blood pressure levels of hypertensive patients increase with the increase of serum CRP concentration.

At present, CRP detection method on the market mainly include hypersensitive CRP (hsCRP) detection, conventional CRP detection, and full-range CRP detection. Hypersensitive CRP detection is mainly used to diagnose and predict the occurrence and development of cardiovascular events, while conventional CRP detection is mainly used for bacterial infection, various inflammatory processes, tissue necrosis and tissue damage (such as post-operative damage), as well as screening, monitoring, disease evaluation and efficacy judgment during recovery period. Early conventional CRP detection methods are mainly based on immuno-scattering turbidity or immuno-transmitting turbidity methods, with a detection capacity of more than 5 mg/L, but they are difficult to predict the risk of cardiovascular disease due to the lack of high sensitivity. Subsequent research and development provide an immune enhancement turbidimetric method, which analysis sensitivity is greatly improved, the lower limit of detection can reach 0.02mg/L. This hypersensitive CRP detection for low concentrations is called hypersensitive CRP detection. With the continuous innovation and improvement of technology, some detection methods can cover the detection linearity of hypersensitivity and full-range CRP at one time, such as chemiluminescence detection methods and immunofluorescence detection methods. The line width of detection can reach 0.02-100mg/L.

At present, the full-range CRP detection methods usually use the addition of competing free antibodies. For example, US Patent Application Publication No. US 2014/0017712 A1 mentions the use of adding a free monoclonal antibody or an antibody that can compete with coating or labeling. However, such a method increases difficulty in operations such as reagent stability. Chinese Patent Publication No. CN105988003A discloses a method in which the purpose of full-range detection is achieved by using alkali neutralization after acid destruction, but it is still not stable and convenient. CN 105 988 003 discloses a kit and its application for whole course detection of CRP based on a chemiluminescent sandwich immunoassay. CN 103 941 017 discloses a kit for detection of CRP by using ELISA. CN 107 632 163 discloses a kit for detection of CRM based on a chemiluminescent sandwich immunoassay. SK Vashist et al., 2014, Analytical Biochemistry, 456(13), 32-37 discloses an assay for CRP detection in whole blood samples, which is based on a combination of sandwich ELISA with immunomagnetic separation. D Tanne et al., Stroke, 37(7), 1720-1724 discloses the use of a chemiluminescent sandwich immunoassay for determining the level of CRP in samples of citrated plasma. EP 1, 679 516 discloses the use of citric acid in sandwich immunoassays for reducing the Hook effect. Citric acid is used as a reagent for adjusting the pH of the reaction mixture to a value below the pI of the antibody before bringing the complex in contact with the detection antibody.

Therefore, there still exists a need in the art to improve the method of the full-range CRP detection to realize a full-range CRP detection method in a stable and convenient detection mode.

### Contents of the Invention

The purpose of the present invention is to overcome the defects of the prior art and provide a stable and convenient method for full-range detection of C-reactive protein as well as a corresponding kit.

The technical solution of the present invention is as follows:
In one aspect, the present invention provides a kit for full-range detection of C-reactive protein, which comprises:
an M reagent, comprising 0.5~1mg/mL magnetic particles coated with a first antibody, 0.04~0.06% (w/v) surfactant, wherein the surfactant is optionally Tween-20, and 8~12% (w/v) sucrose, its solvent is a phosphate buffer with pH=7.0~8.0; wherein the coating amount of the first antibody is 5~20µg/mg magnetic particles;
an R1 reagent, that is a sample treatment solution, which is a citric acid solution with a concentration of 0.1~1M, pH=3.0~4.0;
an R2 reagent, comprising acridinium ester coated with a secondary antibody, 0.5-1% casein and 0.5-1% bovine serum albumin, its solvent is phosphate buffer with pH=7.0-8.0, wherein the coating amount of the secondary antibody is 0.3-0.9µg/µg acridinium ester;
a pre-excitation solution and an excitation solution, wherein the pre-excitation solution is 1% (w/v) hydrogen peroxide solution, and the excitation solution is 1 mol/L sodium hydroxide solution;
wherein the first antibody and the second antibody are both monoclonal antibodies that can specifically react with C-reactive protein, and the first antibody and the second antibody are directed to different epitopes.

In another aspect, the present invention provides a kit for full-range detection of C-reactive protein, which comprises:
a flat-bottomed plate-type chemiluminescence plate coated with a first antibody, which comprises a plate-type luminescence plate. optionally, a 96-well, 384-well or other plate-type luminescence plate, wherein the coating amount of the first antibody is 100~500ng/well, optionally 500ng/well, the coating buffer is a phosphate buffer with pH=7.0~8.0, the blocking solution is 50mM phosphate buffer with pH of 7.2-7.4 comprising 5-8% (w/v) blocking serum or blocking protein. wherein the blocking serum is optionally calf serum, and 0.02% (w/v) sodium azide;
a sample treatment solution, which is a citric acid solution with a concentration of 0.1~1M, pH=3~4;
a labeling enzyme solution, comprising a secondary antibody labeled with horseradish peroxidase or alkaline phosphatase, and having a labeling amount that 1 mg/mL of the secondary antibody is labeled with horseradish peroxidase or alkaline phosphatase in the same proportion;
a color developing solution: when the labeling enzyme is horseradish peroxidase, the color developing solution comprises a color developing solution A and a color developing solution B, and the color developing solution A is hydrogen peroxide, optionally, wherein the formula of the color developing solution A: 13.6g of sodium acetate, 1.6g of citric acid, 0.3ml of 30% hydrogen peroxide, formulated with distilled water to 500ml, the color developing solution B is o-phenylenediamine, optionally, wherein the formula of the color developing solution B: 0.2 g of disodium ethylenediaminetetraacetate, 0.95g of citric acid, 50ml of glycerol, 9.15g of tetramethylbenzidine, formulated with distilled water to 500ml; when the labeling enzyme is alkaline phosphatase, the color developing solution is a reagent purchased from Xiamen Boson Biotechnology Co., Ltd. with Art. No.: 180309-01;
wherein the first antibody and the second antibody are both monoclonal antibodies that can specifically react with C-reactive protein, and the first antibody and the second antibody are directed to different epitopes;
wherein the first antibody is 10C11 and the second antibody is 14D9-2.

In some embodiments, the pH of the citric acid solution is adjusted by disodium hydrogen phosphate dodecahydrate; preferably, the pH of the citric acid solution is 3.0-3.5; and more preferably, the pH of the citric acid solution is 3.2, 3.3, 3.4 or 3.5.

In other embodiments, the concentration of the citric acid is 0.5 mol/L.

The pre-excitation solution is 1% (w/v) hydrogen peroxide solution, the excitation solution is 1 mol/L sodium hydroxide solution, the first antibody is 10C11, and the second antibody is 14D9-2.

In still other embodiments, the method for preparing the M reagent comprises: the first antibody and the magnetic particles are mixed in 2-morpholineethanesulfonic acid buffer with pH=5.0~6.0, coated at 25-37°C for 1-3h, added with 0.1%~0.5% (w/v) bovine serum albumin phosphate buffer with pH=8.0~9.0 to perform termination for 1~3h, the coated magnetic particles are separated and dispersed in a phosphate buffer with pH=7.0~8.0, then added with 0.04~0.06% (w/v) surfactant, wherein the surfactant is optionally Tween-20; in one embodiment, the surfactant is 0.05% (w/v) Tween-20) and 8~12% (w/v) sucrose, optionally, 10% (w/v) sucrose, to obtain the M reagent;
the method for preparing the R2 reagent comprises: the second antibody and acridinium ester are mixed in a phosphate buffer with pH=8.0~9.0, coated at 25-37°C for 1~3h, and then added with a Tris buffer comprising 0.1%~0.5% (w/v) bovine serum albumin and having pH=8.0~9.0 to perform termination for 1~3h so as to obtain a stock solution, and the stock solution is diluted with a phosphate buffer having pH=7.0~8.0 to 1:100-500 to obtain the R2 reagent.

In other embodiments, the method for preparing the luminescent plate coating source comprises: the coated first antibody is diluted with a phosphate buffer having pH=7.0-8.0 as coating buffer to 100-500ng/well, optionally, 500ng/well, added to the luminescent plate, 100µL per well, incubated at 37°C for 2h or 4°C overnight, the coating buffer is poured out, 200µL of the blocking solution comprising 5-8% (w/v) calf serum and 0.02% (w/v) sodium azide is used for incubation at 37°C for 2h, the liquid in the wells is poured out, the plate is dried and sealed under vacuum with aluminum film, and stored in a dry place at 4°C;
the method for preparing the labeling enzyme solution comprises: the second antibody and horseradish peroxidase or alkaline phosphatase in ratio of 1:1 are mixed and labeled and dialyzed in a carbonate buffer with pH=9.6, and the dialysis buffer is replaced every 4 hours and replaced for three times, the enzyme-labeled secondary antibody is collected to be a stock solution, and then the stock solution is diluted with an enzyme diluent purchased from Beijing Wantai Biopharmaceutical Co., Ltd. with Cat. No. ED-11 to 1:500 to obtain the labeling enzyme solution.

In yet another aspect, the present invention provides a method for full-range detection of C-reactive protein, which is performed using the kit of the present invention, and which comprises:
(1) 20µL of a sample is taken and added to 100µL of the R1 reagent to treat the sample, wherein the sample is serum;
(2) 50µL of the M reagent is then added and incubated together for 15min;
(3) after step (2), washing is performed with a phosphate buffer comprising 0.05~0.08% (w/v) Tween-20, then 50 µL of the R2 reagent is added and incubated for 10 minutes;
(4) after step (3), washing is performed with a phosphate buffer comprising 0.05~0.08% (w/v) Tween-20, and 100 µL of the pre-excitation solution is added to perform pre-excitation;
(5) the pre-excitation solution is removed, 100 µL of the excitation solution is then added to perform excitation and detection.

In some embodiments, the citric acid solution is a citric acid solution with a concentration of 0.1~1M, pH=3~4; preferably, the pH of the citric acid solution is adjusted by disodium hydrogen phosphate dodecahydrate, more preferably, the pH of the citric acid solution is 3.0-3.5, more preferably, the pH of the citric acid solution is 3.2, 3.3, 3.4 or 3.5.

In one aspect, the present invention provides a kit for full-range detection of C-reactive protein (direct chemiluminescence, that is, magnetic particle-chemiluminescence method), which comprises the following components:
an M reagent, comprising 0.5~1mg/mL magnetic particles coated with a first antibody, 0.04~0.06% (w/v) surfactant, wherein the surfactant is optionally Tween-20, and 8~12% (w/v) sucrose, its solvent is a phosphate buffer with pH=7.0~8.0; wherein the coating amount of the first antibody is 5~20µg/mg magnetic particles;
an R1 reagent, that is a sample treatment solution, which is a citric acid solution with a concentration of 0.1~1M, pH=3.0~4.0;
an R2 reagent, comprising acridinium ester coated with a secondary antibody, 0.5-1% casein and 0.5-1% bovine serum albumin, its solvent is phosphate buffer with pH=7.0-8.0, wherein the coating amount of the secondary antibody is 0.3-0.9µg/µg acridinium ester;
a pre-excitation solution and an excitation solution, wherein the pre-excitation solution is 1% (w/v) hydrogen peroxide solution, and the excitation solution is 1 mol/L sodium hydroxide solution;
wherein the first antibody and the second antibody are both monoclonal antibodies that can specifically react with C-reactive protein, and the first antibody and the second antibody are directed to different epitopes.

The luminescence mechanism of acridine compounds is: in an alkaline hydrogen peroxide solution, the molecule of acridine compound is attacked by hydrogen peroxide ions to form an unstable peroxy compound, which decomposes into CO₂ and electronically excited N-methyl-acridone, when it returns to its ground state, it emits a photon with a maximum emission wavelength of 430 nm. Surfactants such as Triton X-100, Tween-20, CTAC (hexadecyltrimethylammonium chloride, a cationic surfactant) can enhance luminescence.

In a preferred embodiment of the present invention, the R1 reagent is 0.5M citric acid solution, pH=3~3.5.

Further preferably, the pH of the R1 reagent is adjusted by disodium hydrogen phosphate dodecahydrate.

In one embodiment, the pH of the citric acid solution is adjusted by disodium hydrogen phosphate dodecahydrate. Preferably, the pH of the citric acid solution is 3.0-3.5, and more preferably, the pH of the citric acid solution is 3.2, 3.3, 3.4 or 3.5.

In yet another embodiment, the concentration of the citric acid is 0.5 mol/L.

Further preferably, the M reagent contains 0.05% Tween-20 and 10% sucrose.

Further preferably, the method for preparing the M reagent comprises: the first antibody and the magnetic particles are mixed in 2-morpholineethanesulfonic acid buffer with pH=5.0~6.0, coated at 25-37°C for 1-3h, added with 0.1%~0.5% (w/v) bovine serum albumin phosphate buffer with pH=8.0~9.0 to terminate the coating for 1~3h, the coated magnetic particles are separated and dispersed in a phosphate buffer with pH=7.0~8.0, then added with Tween-20 and sucrose to obtain the M reagent.

Further preferably, the method for preparing the R2 reagent comprises: the second antibody and acridinium ester are mixed in a phosphate buffer with pH=8.0~9.0, coated at 25-37°C for 1~3h, and then added with a Tris buffer comprising 0.1%~0.5% (w/v) bovine serum albumin and having pH=8.0~9.0 to terminate the coating for 1~3h so as to obtain a stock solution, and the stock solution is diluted with a phosphate buffer having pH=7.0~8.0 to 1:100-500 to obtain the R2 reagent.

Further preferably, the pre-excitation solution is a 1% (w/v) hydrogen peroxide solution.

Furthermore, the excitation solution is 1 mol/L sodium hydroxide solution.

In another aspect, the present invention provides a kit for full-range detection of C-reactive protein, such as enzymatic chemiluminescence, namely horseradish peroxidase or alkaline phosphatase plate-type chemiluminescence, which comprises the following components:
a flat-bottomed chemiluminescence plate coated with a first antibody, which comprises a plate-type luminescence plate, optionally, 96-well, 384-well or other plate-type luminescence plate, wherein the coating amount of the first antibody is 100~500ng/well, optionally 500ng/well, the coating buffer is a phosphate buffer with pH=7.0~8.0, the blocking solution is 50mM phosphate buffer with pH of 7.2-7.4 comprising 5-8% (w/v) blocking serum or blocking protein, wherein the blocking serum is optionally calf serum, and 0.02% (w/v) sodium azide;
a sample treatment solution, which is a citric acid solution with a concentration of 0.1~1M, pH=3~4;
a labeling enzyme solution, comprising a secondary antibody labeled with horseradish peroxidase or alkaline phosphatase, and having a labeling amount that 1 mg/mL of the secondary antibody is labeled with horseradish peroxidase or alkaline phosphatase in the same proportion;
a color developing solution: when the labeling enzyme is horseradish peroxidase, the color developing solution comprises a color developing solution A and a color developing solution B, and the color developing solution A is hydrogen peroxide (13.6g of sodium acetate, 1.6g of citric acid, 0.3ml of 30% hydrogen peroxide, formulated with distilled water to 500ml), the color developing solution B is o-phenylenediamine, wherein the formula of the color developing solution B: 0.2 g of disodium ethylenediaminetetraacetate, 0.95g of citric acid, 50ml of glycerol, 9.15g of tetramethylbenzidine, formulated with distilled water to 500ml; when the labeling enzyme is alkaline phosphatase, the color developing solution is a reagent with Art. No.: 180309-01, purchased from: Xiamen Boson Biotechnology Co., Ltd..

Detection: an automatic chemiluminescence analyzer (purchased from: Yantai Addcare Biotechnology Co., Ltd.) is used for reading the luminescence values.

The above-mentioned first antibody and second antibody are both monoclonal antibodies that can specifically react with C-reactive protein.

In a preferred embodiment of the present invention, the sample treatment solution is a 0.5 M citric acid solution with a pH of 3 to 3.5.

Further preferably, the pH of the sample treatment solution is adjusted by disodium hydrogen phosphate dodecahydrate.

In one embodiment, the pH of the citric acid solution is adjusted by disodium hydrogen phosphate dodecahydrate. Preferably, the pH of the citric acid solution is 3.0-3.5, and more preferably, the pH of the citric acid solution is 3.2, 3.3, 3.4 or 3.5.

In yet another embodiment, the concentration of the citric acid is 0.5 mol/L.

Further preferably, the flat-bottomed chemiluminescent plate coated with the first antibody comprises 5-8% calf serum and 0.02% sodium azide.

Further preferably, the method for preparing the flat-bottomed chemiluminescent plate coated with the first antibody comprises: the coated first antibody is diluted with a phosphate buffer having pH=7.0-8.0 as coating buffer to 5µg/mL, i.e., 500ng/well, added to the luminescent plate, 100µL per well, incubated at 37°C for 2h or 4°C overnight, the coating buffer is poured out, 200µL of the blocking solution (5-8% (w/v) calf serum and 0.02% (w/v) sodium azide) is used for incubation at 37°C for 2h, the liquid in the wells is poured out, the plate is dried and sealed under vacuum with aluminum film, and stored in a dry place at 4°C.

Further preferably, the method for preparing the labeling enzyme solution comprises: the second antibody and horseradish peroxidase or alkaline phosphatase in ratio of 1:1 are mixed and labeled and dialyzed in a carbonate buffer with pH=9.6, and the dialysis buffer is replaced every 4 hours, for three times, the enzyme-labeled secondary antibody is collected to be the stock solution, and then the stock solution is diluted with an enzyme diluent purchased from Beijing Wantai Biopharmaceutical Co., Ltd. with Cat. No. ED-11 to 1:500 to obtain the labeling enzyme solution.

Further preferably, when the labeling enzyme is horseradish peroxidase, the color developing solution A is hydrogen peroxide, and the color developing solution B is o-phenylenediamine; when the labeling enzyme is alkaline phosphatase, the color developing solution is a reagent purchased from Xiamen Boson Biotechnology Co., Ltd. with Art. No.: 180309-01.

Furthermore, the content is directly determined by an automatic chemiluminescence analyzer.

The beneficial effect of the present invention is that the detection range of the kit of the present invention can be 0.02mg/L to 100mg/L after a sample treatment solution (citric acid solution with a concentration of 0.1~1M, pH=3~4) is added in one step during the reaction process of the kit of the present invention, so that the kit can meet the requirements of full-range detection of C-reactive protein.

### Brief Description of the Drawings

Figure 1 shows a paired dose-response curve, which is a curve of a calibrator for pairing detection. The left image represents the paired dose-response curve for 10C11-7D9, and the right image represents the paired dose-response curve for 10C11-14D9-2.
Figure 2 shows a correlation analysis of pairing detection results, which evaluates the correlation between samples and background values. The left image represents the correlation analysis of pairing detection results for 10C11-7D9, and the right image represents the correlation analysis of pairing detection results for 10C11-14D9-2.

### Detailed Embodiments of the Invention

The technical solution of the present invention is further illustrated and described below through specific embodiments.

The reagents were of analytical grade, and unless otherwise specified, they were purchased from Xiamen Xilong Chemical Co., Ltd.

In one embodiment, the kit for full-range detection of C-reactive protein (direct chemiluminescence, that is, magnetic particle chemiluminescence method) of the present invention comprises the following components:
an M reagent, comprising 0.8mg/mL magnetic particles coated with a first antibody, 0.05% Tween-20 and 10% sucrose, and its solvent was a phosphate buffer with pH=7.5, wherein the coating amount of the first antibody was 12 µg/µg magnetic particles, the magnetic particles were purchased from Thermo Fisher Scientific and were nano-scale superparamagnetic particles with Fe₃O₄ core. The method for preparing the M reagent comprised: the first antibody and magnetic particles were mixed in 2-morpholineethanesulfonic acid buffer with pH=5.5, coated at 32°C for 1~3h, and added with a phosphate buffer solution comprising 0.3% bovine serum albumin and having pH=8.5 to terminate the coating for 2h, the coated magnetic particles were separated and dispersed in a phosphate buffer solution with pH=7.5, and then added with Tween-20 and sucrose to obtain the M reagent;
an R1 reagent, a citric acid solution with a concentration of 0.5M, pH=3.2, adjusted with disodium hydrogen phosphate dodecahydrate;
an R2 reagent, comprising acridinium ester coated with the secondary antibody, 0.8% casein and 0.8% bovine serum albumin, its solvent was a phosphate buffer with pH=7.5, and the coating amount of the secondary antibody was 12µg/µg acridinium ester. The method for preparing the R2 reagent comprised: the second antibody and acridinium ester were mixed in a phosphate buffer with pH=8.5, coated at 32°C for 1~3h, added with a Tris buffer solution comprising 0.3% bovine serum albumin and having pH=8.5 to terminate the coating for 2h so as to obtain a stock solution; the stock solution was diluted with a phosphate buffer having pH=7.5 to 1:300 so as to obtain the R2 reagent;
pre-excitation solution, 1% (w/v) hydrogen peroxide solution;
exciting solution, 1mol/L sodium hydroxide solution;
the above-mentioned first antibody and second antibody were monoclonal antibodies that could specifically react with C-reactive protein. The first antibody was 10C11 and the second antibody was 14D9-2, both of which were purchased from Xiamen Innovax Biotech CO., Ltd.

The detection method using the above-mentioned kit for full-range detection of C-reactive protein comprised the following steps:
(1) 20µL of a sample (the sample is a serum or a standard C-reactive protein for preparing antigen standard curve) was taken and added to 100µL of the R1 reagent to treat the sample, wherein the sample is serum;
(2) 50µL of the M reagent was then added and incubated together for 15min;
(3) after step (2), washing was performed with a phosphate buffer comprising 0.05% Tween-20, then 50 µL of the R2 reagent was added and incubated for 10min;
(4) after step (3), washing was performed with a phosphate buffer comprising 0.05~0.08% (w/v) Tween-20, and 100 µL of the pre-excitation solution is added to perform pre-excitation;
(5) the pre-excitation solution is removed, 100 µL of the excitation solution is added to perform excitation and detection.

In another embodiment, the kit for full-range detection of C-reactive protein (enzymatic chemiluminescence, that is, horseradish peroxidase plate-type chemiluminescence) of the present invention comprised the following components:
a luminescent plate coating source, comprising a 96-well plate luminescent plate, in which the coating amount of the first antibody was 500ng/well, the coating buffer was a phosphate buffer with pH=7.5, and the blocking solution was 50 mM phosphate buffer with a pH of 7.3 comprising 6% calf serum and 0.02% sodium azide. The method for preparing the luminescent plate coating source comprised: the first antibody was diluted with phosphate buffer having pH=7.5 as coating buffer to 5µg/mL (i.e., 500ng/well), added to the luminescent plate, 100µL per well, incubated at 37°C for 2h or at 4°C overnight, the coating buffer was poured out, 200µL of the blocking solution comprising 6% calf serum and 0.02% sodium azide was used for incubation at 37°C for 2h, the liquid in the wells was poured out, the plate was dried and sealed under vacuum with aluminum film, and stored in a dry place at 4°C;
a sample treatment solution, which is a citric acid solution with a concentration of 0.5M, pH=3.2, adjusted with disodium hydrogen phosphate dodecahydrate;
a labeling enzyme solution, comprising a secondary antibody labeled with horseradish peroxidase or alkaline phosphatase, and having a labeling amount that 1 mg/mL of the secondary antibody was labeled with horseradish peroxidase and alkaline phosphatase in the same proportion. The method for preparing the labeling enzyme solution comprised: the second antibody and horseradish peroxidase or alkaline phosphatase in ratio of 1:1 were mixed and labeled and dialyzed in a carbonate buffer of pH=9.6, and the dialysis buffer was replaced once every 4 hours, for 3 times, the enzyme-labeled secondary antibody was collected to obtain a stock solution, and then the stock solution was diluted with an enzyme diluent with Cat. No. ED-11, purchased from Beijing Wantai Biopharmaceutical Co., Ltd. to 1:500 to obtain the labeling enzyme solution;
a color developing solution: when the labeling enzyme was horseradish peroxidase, the color developing solution A was hydrogen peroxide, and the color developing solution B was o-phenylenediamine; when the labeling enzyme was alkaline phosphatase, the color developing solution was a purchased reagent Xiamen Boson Biotechnology Co., Ltd. with Art. No.: 180309-01.

Detection: an automatic chemiluminescence analyzer (purchased from: Yantai Addcare Biotechnology Co., Ltd.) was used for reading the luminescence values.

The above-mentioned first antibody and second antibody were monoclonal antibodies that could specifically react with C-reactive protein. The first antibody was 10C11 and the second antibody was 14D9-2, both of which were purchased from Xiamen Innovax Biotech CO., Ltd.

The detection method using the above-mentioned kit for full-range detection of C-reactive protein comprised the following steps:
(1) 20µL of a sample was taken and added to 100µL of the sample treatment solution to treat the sample;
(2) then added to the luminescent plate coating source and incubated together at 37°C for 40min;
(3) after step (2), washing was performed for 5 times with a phosphate buffer comprising 0.05% Tween-20, the luminescent plate was turned upside-down till dry, then 100 µL of labeling enzyme solution was added and incubated at 37°C for 40 min;
(4) after step (3), washing was performed for 5 times with a phosphate buffer comprising 0.05% Tween-20, the luminescent plate was turn upside-down till dry; if the labeling enzyme was horseradish peroxidase, 50µL of the color developing solution A and 50µL of the color developing solution B were added, reacted at room temperature for 5min; if the labeling enzyme was alkaline phosphatase, 100µL of color developing solution purchased from: Xiamen Boson Biotechnology Co., Ltd. with Art. No.: 180309-01 was added and reacted at room temperature for 5min; finally, the automatic chemiluminescence analyzer was used to perform detection and read the luminescence values.

### Examples

### Example 1

0.1M citric acid and 0.1M glycine of different pH values were selected respectively as treatment solution, and added to the enzyme immunoassay system (the pH range was 2-6) to evaluate the gradiently diluted C-reactive protein antigen. The relative OD values were shown in Tables 1 and 2 below.

**Table 1: Effects of 0.1M citric acid treatment solutions with different pH values on the detection of C-reactive protein**

| Concentration (mg/L) | pH=2 | pH=3 | pH=4 | pH=5 | pH=6 |
|---|---|---|---|---|---|
| 100.00 | 0.4090 | 1.4410 | 3.7590 | 0.7350 | 1.0130 |
| 25.00 | 0.5760 | 1.3740 | 3.7620 | 1.0160 | 1.2900 |
| 6.25 | 0.1510 | 0.8930 | 3.7590 | 1.3460 | 1.5250 |
| 1.56 | 0.0430 | 0.3720 | 3.7760 | 2.4110 | 2.8010 |
| 0.39 | 0.0120 | 0.0960 | 3.0050 | 1.5960 | 3.2060 |
| 0.10 | 0.0060 | 0.0240 | 0.9480 | 0.8620 | 2.2130 |
| 0.02 | 0.0100 | 0.0110 | 0.2560 | 0.0640 | 0.2760 |

**Table 2: Effects of 0.1M glycine treatment solutions with different pH values on the detection of C-reactive protein**

| Concentration (mg/L) | pH=2 | pH=3 | pH=4 | pH=5 | pH=6 |
|---|---|---|---|---|---|
| 100 | 3.7910 | 3.7500 | 2.6820 | 3.2820 | 1.7660 |
| 25 | 2.6980 | 3.5760 | 2.3680 | 2.9670 | 2.1690 |
| 6.25 | 0.5270 | 3.0060 | 2.5510 | 3.2550 | 2.3350 |
| 1.56 | 0.1100 | 0.6840 | 2.6910 | 3.3620 | 2.8260 |
| 0.39 | 0.0330 | 0.1780 | 1.1610 | 1.7100 | 1.8980 |
| 0.1 | 0.0190 | 0.0440 | 0.5440 | 0.7650 | 0.6870 |
| 0.02 | 0.0140 | 0.0150 | 0.1090 | 0.1850 | 0.0880 |

Table 1 and Table 2 showed the detection results of the traced antigen of the full-range detection of C-reactive protein in the enzyme immunoassay system when the treatment solution was 0.1M citric acid with different pH values (adjusted to different pH values with disodium hydrogen phosphate dodecahydrate) and the treatment solution was 0.1M glycine with different pH values. The results showed that there was an obvious trend in the detection between pH 3-4, while other pH ranges were not ideal. It could be seen from Tables 1 and 2 that when the range of pH 3-4 was selected as the treatment pH of the treatment solution, the sample detection exhibited a tend from high to low, which was better than other pH ranges. However, the line width of the enzyme immunoassay system was not sufficient for full-range detection, so it was considered that the optimal pH range was 3-4 for chemiluminescence platform exploration, and citric acid was used for subsequent experiments.

### Example 2

Based on Example 1, the improvement and adjustment of citric acid concentration (citric acid concentration of 0.1M, 0.5M, 1M) and pH range (pH3 and pH3.5, pH4) were carried out, the relative linear width of the enzyme immunoassay system was relatively narrow, and the preferred solution was adjusted on chemiluminescence platform. The citric acid solutions with different molar concentrations and pH 3-4 were selected as the treatment solution and added to the chemiluminescence detection system (i.e., magnetic particle chemiluminescence platform) to evaluate the gradiently diluted C-reactive protein antigen. By using citric acid with different pH and different concentration, the gradiently diluted C-reactive protein antigen was treated, and the magnetic particle chemiluminescence platform or the enzymatic horseradish peroxidase chemiluminescence platform was used for detection to obtain the detection results, and the obtained results were made into standard curves. Then, the relative luminescence intensities of the 18 clinical samples collected (from Xiamen Zhongshan Affiliated Hospital and Xijing Hospital) were separately shown in Table 3 and Table 4 below.

**Table 3: Correlation of the influence of citric acid treatment solution with different pH and different concentration on the detection of C-reactive protein (magnetic particle chemiluminescence system)**

| | | | | | | | | | | |
|---|---|---|---|---|---|---|---|---|---|---|
| Citric acid concentration (mol/L) | | 0.1 | | | 0.5 | | | 1 | | |
| pH value | | 3 | 3.5 | 4 | 3 | 3.5 | 4 | 3 | 3.5 | 4 |

| Serum serial number of Zhongshan Hospital | Background value (mg/L) | Detection value (mg/L) | | | | | | | | |
|---|---|---|---|---|---|---|---|---|---|---|
| 1257 | 46.70 | 58.17 | 50.68 | 19.67 | 38.70 | 49.77 | 96.64 | 65.72 | 73.63 | 163.59 |
| 1203 | 54.30 | 67.65 | 62.15 | 25.76 | 37.36 | 57.59 | 98.96 | 95.94 | 44.42 | 319.43 |
| 1444 | 170.00 | 106.50 | 120.46 | 37.40 | 87.46 | 137.30 | 189.87 | 273.05 | 209.85 | 79.81 |
| 1315 | 183.00 | 144.27 | 146.56 | 44.94 | 140.15 | 181.43 | 257.77 | 373.53 | 470.72 | 342.03 |
| 1432 | 136.00 | 78.22 | 89.66 | 22.38 | 76.33 | 102.71 | 132.89 | 182.84 | 200.50 | 88.86 |
| 1333 | 110.00 | 99.11 | 100.68 | 45.29 | 62.90 | 109.99 | 186.08 | 165.51 | 65.75 | 186.25 |
| 1478 | 70.30 | 46.69 | 56.99 | 10.28 | 41.24 | 47.21 | 83.52 | 65.98 | 132.74 | 152.31 |
| 1233 | 62.30 | 53.84 | 60.32 | 16.85 | 44.19 | 54.97 | 102.16 | 88.10 | 104.26 | 154.66 |
| 1207 | 84.80 | 97.89 | 75.66 | 17.15 | 79.43 | 69.73 | 113.89 | 107.25 | 120.61 | 91.72 |
| 1210 | 40.10 | 43.45 | 43.21 | 10.23 | 29.06 | 36.09 | 71.60 | 46.09 | 63.54 | 67.85 |
| 1231 | 36.90 | 60.52 | 59.45 | 45.53 | 37.01 | 49.67 | 99.61 | 77.69 | 17.84 | 193.95 |
| 1338 | 33.70 | 39.83 | 39.97 | 6.90 | 30.62 | 35.97 | 69.69 | 47.56 | 5.99 | 124.40 |
| 1223 | 24.20 | 30.60 | 32.36 | 3.34 | 23.25 | 31.46 | 82.90 | 40.27 | 6.61 | 72.51 |
| 1230 | 29.00 | 28.35 | 32.70 | 12.25 | 22.82 | 26.86 | 60.01 | 38.28 | 27.95 | 106.24 |
| 1349 | 16.80 | 19.46 | 24.65 | 8.17 | 15.58 | 19.75 | 56.57 | 29.44 | 12.92 | 88.97 |
| 1317 | 12.10 | 16.12 | 20.92 | 12.24 | 12.03 | 16.52 | 37.35 | 20.29 | 16.66 | 40.57 |
| 1316 | 5.40 | 9.94 | 6.74 | 12.49 | 6.26 | 7.82 | 19.74 | 18.29 | 23.27 | 2.77 |
| 1324 | 9.10 | 10.52 | 11.43 | 17.12 | 8.79 | 7.84 | 24.26 | 13.17 | 19.98 | 39.97 |

| Serum serial number of Zhongshan Hospital | Background value (Log10) | Detection value (Log10) | | | | | | | | |
|---|---|---|---|---|---|---|---|---|---|---|
| 1257 | 1.67 | 1.76 | 1.70 | 1.29 | 1.59 | 1.70 | 1.99 | 1.82 | 1.87 | 2.21 |
| 1203 | 1.73 | 1.83 | 1.79 | 1.41 | 1.57 | 1.76 | 2.00 | 1.98 | 1.65 | 2.50 |
| 1444 | 2.23 | 2.03 | 2.08 | 1.57 | 1.94 | 2.14 | 2.28 | 2.44 | 2.32 | 1.90 |
| 1315 | 2.26 | 2.16 | 2.17 | 1.65 | 2.15 | 2.26 | 2.41 | 2.57 | 2.67 | 2.53 |
| 1432 | 2.13 | 1.89 | 1.95 | 1.35 | 1.88 | 2.01 | 2.12 | 2.26 | 2.30 | 1.95 |
| 1333 | 2.04 | 2.00 | 2.00 | 1.66 | 1.80 | 2.04 | 2.27 | 2.22 | 1.82 | 2.27 |
| 1478 | 1.85 | 1.67 | 1.76 | 1.01 | 1.62 | 1.67 | 1.92 | 1.82 | 2.12 | 2.18 |
| 1233 | 1.79 | 1.73 | 1.78 | 1.23 | 1.65 | 1.74 | 2.01 | 1.94 | 2.02 | 2.19 |
| 1207 | 1.93 | 1.99 | 1.88 | 1.23 | 1.90 | 1.84 | 2.06 | 2.03 | 2.08 | 1.96 |
| 1210 | 1.60 | 1.64 | 1.64 | 1.01 | 1.46 | 1.56 | 1.85 | 1.66 | 1.80 | 1.83 |
| 1231 | 1.57 | 1.78 | 1.77 | 1.66 | 1.57 | 1.70 | 2.00 | 1.89 | 1.25 | 2.29 |
| 1338 | 1.53 | 1.60 | 1.60 | 0.84 | 1.49 | 1.56 | 1.84 | 1.68 | 0.78 | 2.09 |
| 1223 | 1.38 | 1.49 | 1.51 | 0.52 | 1.37 | 1.50 | 1.92 | 1.61 | 0.82 | 1.86 |
| 1230 | 1.46 | 1.45 | 1.51 | 1.09 | 1.36 | 1.43 | 1.78 | 1.58 | 1.45 | 2.03 |
| 1349 | 1.23 | 1.29 | 1.39 | 0.91 | 1.19 | 1.30 | 1.75 | 1.47 | 1.11 | 1.95 |
| 1317 | 1.08 | 1.21 | 1.32 | 1.09 | 1.08 | 1.22 | 1.57 | 1.31 | 1.22 | 1.61 |
| 1316 | 0.73 | 1.00 | 0.83 | 1.10 | 0.80 | 0.89 | 1.30 | 1.26 | 1.37 | 0.44 |
| 1324 | 0.96 | 1.02 | 1.06 | 1.23 | 0.94 | 0.89 | 1.38 | 1.12 | 1.30 | 1.60 |
| **r²** | | 0.9304 | 0.9586 | 0.2907 | **0.9692** | **0.9615** | 0.9179 | 0.9251 | 0.5942 | 0.4972 |

**Table 4: Correlation of the influence of citric acid treatment solution with different pH and different concentration on the detection of C-reactive protein (enzymatic horseradish peroxidase chemiluminescence system)**

| | | | | | | | | | | |
|---|---|---|---|---|---|---|---|---|---|---|
| Citric acid concentration (mol/L) | | 0.1 | | | 0.5 | | | 1 | | |
| pH value | | 3 | 3.5 | 4 | 3 | 3.5 | 4 | 3 | 3.5 | 4 |

| Serum serial number of Zhongshan Hospital | Background value (mg/L) | Detection value (mg/L) | | | | | | | | |
|---|---|---|---|---|---|---|---|---|---|---|
| 1242 | 20 | 27.77 | 139.53 | 37.45 | 37.13 | 17.22 | 140.83 | 82.22 | 92.16 | 9.14 |
| 1351 | 5 | 34.19 | 163.47 | 20.06 | 13.03 | 3.15 | 53.52 | 25.86 | 34.85 | 5.10 |
| 1402 | 3.8 | 15.56 | 89.20 | 21.63 | 9.55 | 3.51 | 37.49 | 24.61 | 31.38 | 1.56 |
| 1417 | 25.8 | 59.31 | 289.25 | 30.77 | 54.35 | 15.76 | 221.95 | 73.38 | 110.76 | 7.75 |
| 1408 | 33.2 | 59.31 | 348.65 | 43.69 | 66.34 | 24.84 | 136.92 | 133.23 | 151.15 | 10.03 |
| 1341 | 38.1 | 76.55 | 501.37 | 37.72 | 71.94 | 34.57 | 68.66 | 159.38 | 352.94 | 10.32 |
| 1255 | 45.7 | 64.17 | 234.53 | 42.15 | 83.93 | 39.37 | 137.79 | 195.34 | 231.96 | 11.19 |
| 1247 | 55.6 | 51.16 | 142.44 | 23.93 | 91.89 | 27.36 | 121.47 | 97.02 | 82.81 | 11.30 |
| 1276 | 62.3 | 82.81 | 302.86 | 22.94 | 94.97 | 55.71 | 92.95 | 206.66 | 386.77 | 12.87 |
| 1365 | 65.4 | 64.67 | 406.95 | 34.06 | 113.07 | 47.36 | 129.91 | 303.71 | 240.53 | 12.23 |
| 1404 | 140 | 104.87 | 405.49 | 87.16 | 209.22 | 73.61 | 132.60 | 239.11 | 264.62 | 15.46 |
| 1246 | 13.2 | 27.49 | 125.45 | 27.00 | 19.99 | 5.67 | 125.97 | 54.08 | 60.48 | 9.04 |
| 1239 | 14.6 | 42.23 | 125.20 | 21.44 | 20.84 | 8.52 | 130.21 | 52.57 | 123.92 | 6.15 |
| 1382 | 133 | 85.53 | 592.49 | 70.10 | 155.30 | 90.13 | 100.33 | 424.02 | 506.85 | 19.91 |
| 1393 | 136 | 83.84 | 444.01 | 32.82 | 211.47 | 69.50 | 92.66 | 163.08 | 187.86 | 18.68 |
| 1484 | 70.3 | 59.54 | 970.42 | 102.66 | 105.58 | 43.79 | 85.37 | 421.22 | 383.87 | 21.00 |
| 1277 | 18.4 | 32.57 | 91.97 | 56.87 | 29.44 | 12.78 | 168.98 | 110.16 | 115.56 | 8.94 |
| 1493 | 67.7 | 72.98 | 336.93 | 307.01 | 104.80 | 59.61 | 74.96 | 393.77 | 340.69 | 17.50 |

| Serum serial number of Zhongshan Hospital | Background value (Log10) | Detection value (Log10) | | | | | | | | |
|---|---|---|---|---|---|---|---|---|---|---|
| 1242 | 1.30 | 1.44 | 2.14 | 1.57 | 1.57 | 1.24 | 2.15 | 1.91 | 1.96 | 0.96 |
| 1351 | 0.70 | 1.53 | 2.21 | 1.30 | 1.11 | 0.50 | 1.73 | 1.41 | 1.54 | 0.71 |
| 1402 | 0.58 | 1.19 | 1.95 | 1.34 | 0.98 | 0.54 | 1.57 | 1.39 | 1.50 | 0.19 |
| 1417 | 1.41 | 1.77 | 2.46 | 1.49 | 1.74 | 1.20 | 2.35 | 1.87 | 2.04 | 0.89 |
| 1408 | 1.52 | 1.77 | 2.54 | 1.64 | 1.82 | 1.40 | 2.14 | 2.12 | 2.18 | 1.00 |
| 1341 | 1.58 | 1.88 | 2.70 | 1.58 | 1.86 | 1.54 | 1.84 | 2.20 | 2.55 | 1.01 |
| 1255 | 1.66 | 1.81 | 2.37 | 1.62 | 1.92 | 1.60 | 2.14 | 2.29 | 2.37 | 1.05 |
| 1247 | 1.75 | 1.71 | 2.15 | 1.38 | 1.96 | 1.44 | 2.08 | 1.99 | 1.92 | 1.05 |
| 1276 | 1.79 | 1.92 | 2.48 | 1.36 | 1.98 | 1.75 | 1.97 | 2.32 | 2.59 | 1.11 |
| 1365 | 1.82 | 1.81 | 2.61 | 1.53 | 2.05 | 1.68 | 2.11 | 2.48 | 2.38 | 1.09 |
| 1404 | 2.15 | 2.02 | 2.61 | 1.94 | 2.32 | 1.87 | 2.12 | 2.38 | 2.42 | 1.19 |
| 1246 | 1.12 | 1.44 | 2.10 | 1.43 | 1.30 | 0.75 | 2.10 | 1.73 | 1.78 | 0.96 |
| 1239 | 1.16 | 1.63 | 2.10 | 1.33 | 1.32 | 0.93 | 2.11 | 1.72 | 2.09 | 0.79 |
| 1382 | 2.12 | 1.93 | 2.77 | 1.85 | 2.19 | 1.95 | 2.00 | 2.63 | 2.70 | 1.30 |
| 1393 | 2.13 | 1.92 | 2.65 | 1.52 | 2.33 | 1.84 | 1.97 | 2.21 | 2.27 | 1.27 |
| 1484 | 1.85 | 1.77 | 2.99 | 2.01 | 2.02 | 1.64 | 1.93 | 2.62 | 2.58 | 1.32 |
| 1277 | 1.26 | 1.51 | 1.96 | 1.75 | 1.47 | 1.11 | 2.23 | 2.04 | 2.06 | 0.95 |
| 1493 | 1.83 | 1.86 | 2.53 | 2.49 | 2.02 | 1.78 | 1.87 | 2.60 | 2.53 | 1.24 |
| **r²** | | 0.7954 | 0.4898 | 0.2703 | 0.9759 | 0.9495 | 0.1002 | 0.8034 | 0.7264 | 0.8121 |

Tables 3 and 4 showed that when three pH ranges of 3, 3.5 and 4 were fixed and different citric acid concentrations (0.1M, 0.5M and 1M) were used, the luminescent platform was used to detect the traced antigen of the full-range C-reactive protein and evaluate 18 samples. It was found that the citric acid with concentration of 0.5M and pH 3-3.5 showed better results. It could be seen from Table 3 that under the above concentration and pH, the correlation between serums was relative better between pH 3 to 3.5; 0.5M citric acid was preferred, the fluctuation was relatively smaller between pH 3 to 3.5 for 0.5M citric acid, and thus it was considered relatively stable.

### Example 3

0.5M citric acid was selected to further optimize and refine the pH concentration (pH 2.8-4). 0.5M citric acid of different pH was used as the treatment solution to treat the gradiently diluted C-reactive protein antigen, and the magnetic particle chemiluminescence platform or the enzymatic horseradish peroxidase chemiluminescence platform was used for detection to obtain the detection results; and then the results were made into standard curves. The collected 18 clinical samples were then detected, and the detection results were shown in Table 5 and Table 6.

**Table 5 Correlation of the influence of 0.5M citric acid treatment solutions with different pH on the detection of C-reactive protein (magnetic particle chemiluminescence system)**

| | | | | | | | | | |
|---|---|---|---|---|---|---|---|---|---|
| Citric acid concentration (mol/L) | | 0.5 | | | | | | | |
| pH value | | 2.8 | 3.0 | 3.2 | 3.4 | 3.5 | 3.6 | 3.8 | 4 |

| Serum serial number of Zhongshan Hospital | Backgroun d value (mg/L) | Detection value (mg/L) | | | | | | | |
|---|---|---|---|---|---|---|---|---|---|
| 1257 | 46.70 | 41.51 | 38.70 | 34.66 | 34.23 | 49.77 | 39.04 | 39.02 | 96.64 |
| 1203 | 54.30 | 52.57 | 37.36 | 42.20 | 37.53 | 57.59 | 51.86 | 47.60 | 98.96 |
| 1444 | 170.00 | 98.97 | 87.46 | 86.84 | 72.81 | 137.30 | 78.51 | 64.71 | 189.87 |
| 1315 | 183.00 | 180.00 | 140.15 | 136.04 | 110.94 | 181.43 | 113.01 | 81.42 | 257.77 |
| 1432 | 136.00 | 75.33 | 76.33 | 72.26 | 66.97 | 102.71 | 74.05 | 58.84 | 132.89 |
| 1333 | 110.00 | 78.86 | 62.90 | 63.58 | 58.17 | 109.99 | 69.18 | 62.38 | 186.08 |
| 1478 | 70.30 | 51.60 | 41.24 | 39.64 | 39.12 | 47.21 | 40.94 | 44.16 | 83.52 |
| 1233 | 62.30 | 44.84 | 44.19 | 41.87 | 43.44 | 54.97 | 45.01 | 39.10 | 102.16 |
| 1207 | 84.80 | 101.81 | 79.43 | 71.45 | 55.36 | 69.73 | 54.71 | 46.83 | 113.89 |
| 1210 | 40.10 | 29.66 | 29.06 | 27.17 | 26.77 | 36.09 | 37.45 | 36.37 | 71.60 |
| 1231 | 36.90 | 45.18 | 37.01 | 38.04 | 33.17 | 49.67 | 39.54 | 42.19 | 99.61 |
| 1338 | 33.70 | 32.44 | 30.62 | 27.45 | 26.63 | 35.97 | 38.25 | 34.35 | 69.69 |
| 1223 | 24.20 | 23.28 | 23.25 | 21.48 | 22.13 | 31.46 | 20.15 | 37.00 | 82.90 |
| 1230 | 29.00 | 25.81 | 22.82 | 20.91 | 26.46 | 26.86 | 29.64 | 34.47 | 60.01 |
| 1349 | 16.80 | 15.64 | 15.58 | 16.75 | 17.43 | 19.75 | 23.79 | 30.96 | 56.57 |
| 1317 | 12.10 | 10.63 | 12.03 | 13.21 | 15.00 | 16.52 | 21.95 | 23.37 | 37.35 |
| 1316 | 5.40 | 5.91 | 6.26 | 7.11 | 8.13 | 7.82 | 11.94 | 13.43 | 19.74 |
| 1324 | 9.10 | 8.11 | 8.79 | 9.96 | 10.39 | 7.84 | 14.53 | 17.05 | 24.26 |

| Serum serial number of Zhongshan Hospital | Backgroun d value (Log10) | Detection value (Log10) | | | | | | | |
|---|---|---|---|---|---|---|---|---|---|
| 1257 | 1.67 | 1.62 | 1.59 | 1.54 | 1.53 | 1.70 | 1.59 | 1.59 | 1.99 |
| 1203 | 1.73 | 1.72 | 1.57 | 1.63 | 1.57 | 1.76 | 1.71 | 1.68 | 2.00 |
| 1444 | 2.23 | 2.00 | 1.94 | 1.94 | 1.86 | 2.14 | 1.89 | 1.81 | 2.28 |
| 1315 | 2.26 | 2.26 | 2.15 | 2.13 | 2.05 | 2.26 | 2.05 | 1.91 | 2.41 |
| 1432 | 2.13 | 1.88 | 1.88 | 1.86 | 1.83 | 2.01 | 1.87 | 1.77 | 2.12 |
| 1333 | 2.04 | 1.90 | 1.80 | 1.80 | 1.76 | 2.04 | 1.84 | 1.80 | 2.27 |
| 1478 | 1.85 | 1.71 | 1.62 | 1.60 | 1.59 | 1.67 | 1.61 | 1.65 | 1.92 |
| 1233 | 1.79 | 1.65 | 1.65 | 1.62 | 1.64 | 1.74 | 1.65 | 1.59 | 2.01 |
| 1207 | 1.93 | 2.01 | 1.90 | 1.85 | 1.74 | 1.84 | 1.74 | 1.67 | 2.06 |
| 1210 | 1.60 | 1.47 | 1.46 | 1.43 | 1.43 | 1.56 | 1.57 | 1.56 | 1.85 |
| 1231 | 1.57 | 1.65 | 1.57 | 1.58 | 1.52 | 1.70 | 1.60 | 1.63 | 2.00 |
| 1338 | 1.53 | 1.51 | 1.49 | 1.44 | 1.43 | 1.56 | 1.58 | 1.54 | 1.84 |
| 1223 | 1.38 | 1.37 | 1.37 | 1.33 | 1.35 | 1.50 | 1.30 | 1.57 | 1.92 |
| 1230 | 1.46 | 1.41 | 1.36 | 1.32 | 1.42 | 1.43 | 1.47 | 1.54 | 1.78 |
| 1349 | 1.23 | 1.19 | 1.19 | 1.22 | 1.24 | 1.30 | 1.38 | 1.49 | 1.75 |
| 1317 | 1.08 | 1.03 | 1.08 | 1.12 | 1.18 | 1.22 | 1.34 | 1.37 | 1.57 |
| 1316 | 0.73 | 0.77 | 0.80 | 0.85 | 0.91 | 0.89 | 1.08 | 1.13 | 1.30 |
| 1324 | 0.96 | 0.91 | 0.94 | 1.00 | 1.02 | 0.89 | 1.16 | 1.23 | 1.38 |
| **r²** | | 0.9547 | 0.9692 | 0.9672 | 0.9786 | 0.9615 | 0.9471 | 0.9319 | 0.9179 |

**Table 6: Correlation of the influence of 0.5M citric acid treatment solution with different pH on the detection of C-reactive protein (enzymatic horseradish peroxidase chemiluminescence system)**

| | | | | | | | | | |
|---|---|---|---|---|---|---|---|---|---|
| Citric acid concentration (mol/L) | | 0.5 | | | | | | | |
| pH value | | 2.8 | 3.0 | 3.2 | 3.4 | 3.5 | 3.6 | 3.8 | 4 |

| Serum serial number of Zhongshan Hospital | Backgroun d value (mg/L) | Detection value (mg/L) | | | | | | | |
|---|---|---|---|---|---|---|---|---|---|
| 1445 | 170 | 17.65 | 65.14 | 122.17 | 118.01 | 101.83 | 133.13 | 87.17 | 28.26 |
| 1449 | 140 | 18.45 | 58.83 | 120.32 | 138.13 | 139.69 | 137.80 | 113.82 | 22.61 |
| 1283 | 114 | 13.50 | 33.13 | 74.65 | 125.50 | 87.12 | 111.72 | 150.29 | 29.15 |
| 1238 | 114 | 18.20 | 53.46 | 94.57 | 157.07 | 130.74 | 136.98 | 174.20 | 28.40 |
| 1465 | 73.1 | 15.85 | 31.78 | 59.66 | 115.18 | 104.39 | 119.16 | 136.63 | 23.71 |
| 1366 | 65.4 | 8.13 | 23.87 | 45.86 | 83.41 | 54.86 | 58.97 | 86.38 | 22.05 |
| 1303 | 58.8 | 9.23 | 24.63 | 55.07 | 88.79 | 57.88 | 79.32 | 52.87 | 12.73 |
| 1487 | 52.2 | 5.90 | 15.02 | 33.21 | 73.56 | 58.32 | 83.29 | 105.08 | 30.43 |
| 1345 | 42.3 | 3.41 | 10.48 | 18.21 | 33.29 | 38.14 | 37.83 | 65.16 | 28.49 |
| 1454 | 33.2 | 3.67 | 13.92 | 25.27 | 54.27 | 38.88 | 43.03 | 57.80 | 36.47 |
| 1461 | 25.8 | 2.74 | 9.24 | 20.37 | 37.56 | 28.74 | 50.86 | 69.94 | 32.99 |
| 1394 | 18.8 | 3.07 | 11.64 | 20.62 | 42.19 | 26.63 | 33.52 | 82.36 | 33.42 |
| 1470 | 9.2 | 1.70 | 4.39 | 8.60 | 14.50 | 15.27 | 17.11 | 32.14 | 41.04 |
| 1407 | 8.1 | 0.39 | 1.94 | 7.01 | 8.82 | 6.54 | 7.64 | 10.36 | 37.76 |
| 1370 | 5.9 | 0.36 | 1.89 | 4.58 | 11.98 | 6.72 | 6.71 | 6.75 | 27.83 |

| Serum serial number of Zhongshan Hospital | Backgroun d value (Log10) | Detection value (Log10) | | | | | | | |
|---|---|---|---|---|---|---|---|---|---|
| 1445 | 2.23 | 1.25 | 1.81 | 2.09 | 2.07 | 2.01 | 2.12 | 1.94 | 1.45 |
| 1449 | 2.15 | 1.27 | 1.77 | 2.08 | 2.14 | 2.15 | 2.14 | 2.06 | 1.35 |
| 1283 | 2.06 | 1.13 | 1.52 | 1.87 | 2.10 | 1.94 | 2.05 | 2.18 | 1.46 |
| 1238 | 2.06 | 1.26 | 1.73 | 1.98 | 2.20 | 2.12 | 2.14 | 2.24 | 1.45 |
| 1465 | 1.86 | 1.20 | 1.50 | 1.78 | 2.06 | 2.02 | 2.08 | 2.14 | 1.37 |
| 1366 | 1.82 | 0.91 | 1.38 | 1.66 | 1.92 | 1.74 | 1.77 | 1.94 | 1.34 |
| 1303 | 1.77 | 0.97 | 1.39 | 1.74 | 1.95 | 1.76 | 1.90 | 1.72 | 1.10 |
| 1487 | 1.72 | 0.77 | 1.18 | 1.52 | 1.87 | 1.77 | 1.92 | 2.02 | 1.48 |
| 1345 | 1.63 | 0.53 | 1.02 | 1.26 | 1.52 | 1.58 | 1.58 | 1.81 | 1.45 |
| 1454 | 1.52 | 0.56 | 1.14 | 1.40 | 1.73 | 1.59 | 1.63 | 1.76 | 1.56 |
| 1461 | 1.41 | 0.44 | 0.97 | 1.31 | 1.57 | 1.46 | 1.71 | 1.84 | 1.52 |
| 1394 | 1.27 | 0.49 | 1.07 | 1.31 | 1.63 | 1.43 | 1.53 | 1.92 | 1.52 |
| 1470 | 0.96 | 0.23 | 0.64 | 0.93 | 1.16 | 1.18 | 1.23 | 1.51 | 1.61 |
| 1407 | 0.91 | -0.41 | 0.29 | 0.85 | 0.95 | 0.82 | 0.88 | 1.02 | 1.58 |
| 1370 | 0.77 | -0.44 | 0.28 | 0.66 | 1.08 | 0.83 | 0.83 | 0.83 | 1.44 |
| **r²** | | **0.9206** | **0.9468** | **0.9591** | **0.9154** | **0.9401** | **0.9204** | **0.7299** | **0.2197** |

Tables 5 and 6 showed that when the optimal citric acid concentration previously explored was used, the pH concentration was carefully explored, disodium hydrogen phosphate dodecahydrate was selected to adjust different pH values (including pH 2.8 to 4), and the luminescence platform was used to detect the traced antigen of full-range C-reactive protein. It was found that the test samples of pH (3.0-3.5) showed better correlation, in which the magnetic particle chemiluminescence platform was the best at pH 3.4, and showed the best linear detection result.

In the above pH range and in combination with the results in Table 5, the pH was in the range of 3.0 to 3.5, the C-reactive protein single serum had the linear correlation r² of above 0.96, the final preferred condition was 0.5M citric acid with pH (3.4), and the correlation in detection of 18 serum samples was above 0.97. From the results in Table 6, the enzymatic horseradish peroxidase luminescence platform showed the best linear detection results at pH 3.2. The correlation of 15 serum samples was above 0.959.

### Example 4

The optimized detection system was used to detect gradiently diluted C-reactive protein antigen to make a standard curve, then the collected 47 clinical samples were detected, their concentration values were calculated through the standard curve and subjected to the correlation evaluation against the clinical background values, and the results were shown in Table 7 below (magnetic particle chemiluminescence platform):

**Table 7**

| Serum serial number of Zhongshan Hospital | Background value (mg/L) | Log10 | Detection value (mg/L) | Log10 |
|---|---|---|---|---|
| 1 | 10.59 | 1.02 | 10.99 | 1.04 |
| 2 | 16.95 | 1.23 | 14.88 | 1.17 |
| 3 | 107.75 | 2.03 | 112.29 | 2.05 |
| 4 | 23.77 | 1.38 | 16.99 | 1.23 |
| 5 | 11.88 | 1.07 | 12.97 | 1.11 |
| 6 | 37.34 | 1.57 | 27.23 | 1.44 |
| 7 | 14.8 | 1.17 | 15.41 | 1.19 |
| 8 | 65.23 | 1.81 | 71.81 | 1.86 |
| 9 | 64.93 | 1.81 | 49.28 | 1.69 |
| 10 | 135.45 | 2.13 | 225.39 | 2.35 |
| 11 | 38.85 | 1.59 | 25.73 | 1.41 |
| 12 | 81.04 | 1.91 | 102.13 | 2.01 |
| 13 | 37.34 | 1.57 | 21.58 | 1.33 |
| 14 | 15.4 | 1.19 | 11.87 | 1.07 |
| 15 | 10.88 | 1.04 | 12.81 | 1.11 |
| 16 | 15.09 | 1.18 | 16.10 | 1.21 |
| 17 | 6.65 | 0.82 | 6.85 | 0.84 |
| 18 | 9 | 0.95 | 8.20 | 0.91 |
| 19 | 69.29 | 1.84 | 40.24 | 1.60 |
| 20 | 85.55 | 1.93 | 90.29 | 1.96 |
| 21 | 9.42 | 0.97 | 10.08 | 1.00 |
| 22 | 14.11 | 1.15 | 15.54 | 1.19 |
| 23 | 33 | 1.52 | 23.67 | 1.37 |
| 24 | 50.97 | 1.71 | 35.66 | 1.55 |
| 25 | 28.42 | 1.45 | 28.40 | 1.45 |

| Serum serial number of Xijing Hospital | | | | |
|---|---|---|---|---|
| 1 | 1.5 | 0.18 | 2.11 | 0.32 |
| 2 | 1.32 | 0.12 | 2.29 | 0.36 |
| 3 | 4.24 | 0.63 | 5.57 | 0.75 |
| 4 | 0.285 | -0.55 | 0.66 | -0.18 |
| 5 | 0.737 | -0.13 | 1.18 | 0.07 |
| 6 | 5.62 | 0.75 | 6.60 | 0.82 |
| 7 | 3.63 | 0.56 | 4.65 | 0.67 |
| 8 | 1.37 | 0.14 | 2.44 | 0.39 |
| 9 | 1.39 | 0.14 | 2.03 | 0.31 |
| 10 | 37 | 1.57 | 21.02 | 1.32 |
| 11 | 1.4 | 0.15 | 2.49 | 0.40 |
| 12 | 0.159 | -0.80 | 0.34 | -0.46 |
| 13 | 2.87 | 0.46 | 3.94 | 0.60 |
| 14 | 44.3 | 1.65 | 31.67 | 1.50 |
| 15 | 45 | 1.65 | 26.10 | 1.42 |
| 16 | 17.4 | 1.24 | 16.19 | 1.21 |
| 17 | 60.1 | 1.78 | 30.51 | 1.48 |
| 18 | 3.02 | 0.48 | 5.27 | 0.72 |
| 19 | 0.665 | -0.18 | 1.33 | 0.12 |
| 20 | 15.4 | 1.19 | 12.34 | 1.09 |
| 21 | 0.381 | -0.42 | 0.93 | -0.03 |
| 22 | 1.81 | 0.26 | 2.87 | 0.46 |

Through correlation evaluation, the correlation equation of the two was y = 0.8151x + 0.2179, and the correlation coefficient r² = 0.9692, indicating that the two had a good correlation.

The optimized detection system was used to detect gradiently diluted C-reactive protein antigen to make a standard curve, then the collected 73 clinical samples were detected, their concentration values were calculated through the standard curve and subjected to the correlation evaluation against the clinical background values, and the results were shown in Table 8 below (enzymatic horseradish peroxidase chemiluminescence platform):

**Table 8**

| Serum serial number of Zhongshan Hospital | Background value (mg/L) | Log10 | Detection value (mg/L) | Log10 |
|---|---|---|---|---|
| 1 | 20 | 1.30 | 32.42 | 1.51 |
| 2 | 5 | 0.70 | 5.37 | 0.73 |
| 3 | 3.8 | 0.58 | 5.90 | 0.77 |
| 4 | 22.8 | 1.36 | 46.23 | 1.66 |
| 5 | 25.8 | 1.41 | 40.60 | 1.61 |
| 6 | 33.2 | 1.52 | 53.73 | 1.73 |
| 7 | 38.1 | 1.58 | 67.42 | 1.83 |
| 8 | 45.7 | 1.66 | 73.19 | 1.86 |
| 9 | 62.3 | 1.79 | 89.68 | 1.95 |
| 10 | 65.4 | 1.82 | 74.84 | 1.87 |
| 11 | 140 | 2.15 | 155.47 | 2.19 |
| 12 | 110 | 2.04 | 140.33 | 2.15 |
| 13 | 13.2 | 1.12 | 12.42 | 1.09 |
| 14 | 14.6 | 1.16 | 22.56 | 1.35 |
| 15 | 133 | 2.12 | 176.93 | 2.25 |
| 16 | 45 | 1.65 | 93.78 | 1.97 |
| 17 | 65.4 | 1.82 | 111.15 | 2.05 |
| 18 | 70.3 | 1.85 | 92.69 | 1.97 |
| 19 | 18.4 | 1.26 | 27.67 | 1.44 |
| 20 | 67.7 | 1.83 | 82.16 | 1.91 |
| 21 | 170 | 2.23 | 203.55 | 2.31 |
| 22 | 170 | 2.23 | 228.36 | 2.36 |
| 23 | 140 | 2.15 | 216.73 | 2.34 |
| 24 | 182 | 2.26 | 555.17 | 2.74 |
| 25 | 41.7 | 1.62 | 60.82 | 1.78 |
| 26 | 43.1 | 1.63 | 57.47 | 1.76 |
| 27 | 33.2 | 1.52 | 44.20 | 1.65 |
| 28 | 42.3 | 1.63 | 48.68 | 1.69 |
| 29 | 31.3 | 1.50 | 42.16 | 1.62 |
| 30 | 86.1 | 1.94 | 179.86 | 2.25 |
| 31 | 58.8 | 1.77 | 100.40 | 2.00 |
| 32 | 39.6 | 1.60 | 59.01 | 1.77 |
| 33 | 21.8 | 1.34 | 46.29 | 1.67 |
| 34 | 41.7 | 1.62 | 59.99 | 1.78 |
| 35 | 33.3 | 1.52 | 73.34 | 1.87 |
| 36 | 58.6 | 1.77 | 104.54 | 2.02 |
| 37 | 57.8 | 1.76 | 76.28 | 1.88 |
| 38 | 29 | 1.46 | 43.97 | 1.64 |
| 39 | 39.6 | 1.60 | 32.97 | 1.52 |
| 40 | 30.2 | 1.48 | 38.47 | 1.59 |

| Serum serial number of Xijing Hospital | | | | |
|---|---|---|---|---|
| 1 | 58.6 | 1.77 | 110.22 | 2.04 |
| 2 | 26.3 | 1.42 | 28.64 | 1.46 |
| 3 | 24.2 | 1.38 | 22.55 | 1.35 |
| 4 | 5.6 | 0.75 | 7.98 | 0.90 |
| 5 | 7 | 0.85 | 9.95 | 1.00 |
| 6 | 9.1 | 0.96 | 10.88 | 1.04 |
| 7 | 7.8 | 0.89 | 10.32 | 1.01 |
| 8 | 6.9 | 0.84 | 7.85 | 0.89 |
| 9 | 6.5 | 0.81 | 10.58 | 1.02 |
| 10 | 12.1 | 1.08 | 19.77 | 1.30 |
| 11 | 16.8 | 1.23 | 25.96 | 1.41 |
| 12 | 42.3 | 1.63 | 50.42 | 1.70 |
| 13 | 114 | 2.06 | 179.29 | 2.25 |
| 14 | 6.9 | 0.84 | 5.40 | 0.73 |
| 15 | 17.1 | 1.23 | 21.13 | 1.32 |
| 16 | 58.6 | 1.77 | 102.90 | 2.01 |
| 17 | 213 | 2.33 | 418.66 | 2.62 |
| 18 | 20.3 | 1.31 | 35.05 | 1.54 |
| 19 | 170 | 2.23 | 196.84 | 2.29 |
| 20 | 140 | 2.15 | 187.56 | 2.27 |
| 21 | 114 | 2.06 | 152.75 | 2.18 |
| 22 | 114 | 2.06 | 144.88 | 2.16 |
| 23 | 77.9 | 1.89 | 185.85 | 2.27 |
| 24 | 73.1 | 1.86 | 97.58 | 1.99 |
| 25 | 65.4 | 1.82 | 71.60 | 1.85 |
| 26 | 58.8 | 1.77 | 81.52 | 1.91 |
| 27 | 52.2 | 1.72 | 48.59 | 1.69 |
| 28 | 33.2 | 1.52 | 34.49 | 1.54 |
| 29 | 25.8 | 1.41 | 37.18 | 1.57 |
| 30 | 18.8 | 1.27 | 30.58 | 1.49 |
| 31 | 9.2 | 0.96 | 12.20 | 1.09 |
| 32 | 8.1 | 0.91 | 7.77 | 0.89 |
| 33 | 5.9 | 0.77 | 5.73 | 0.76 |

Through correlation evaluation, the correlation equation between the two was y = 1.056x + 0.0619, and the correlation coefficient r² = 0.9506, indicating that the two had a good correlation.

### Example 5

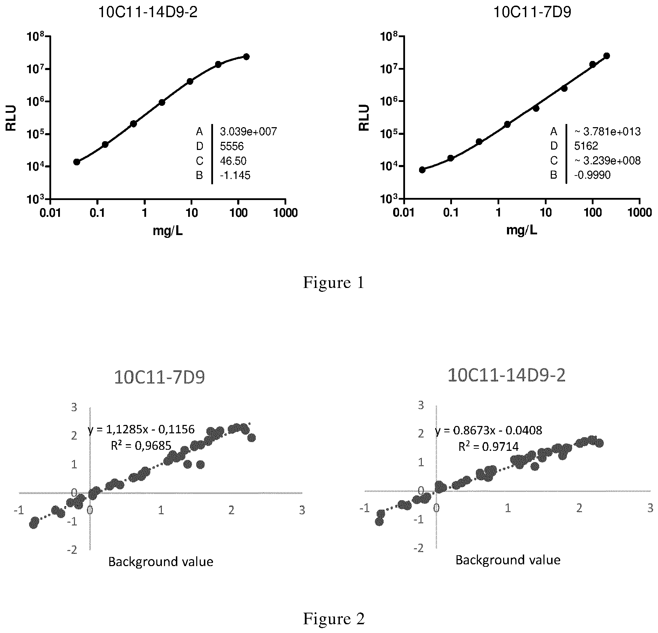

The optimized detection system and the reagents for acid-treatment and alkali-neutralization as mentioned in the patent application with publication number CN105988003A were used to detect the gradiently diluted C-reactive protein antigen so as to make standard curves, and then the collected 48 clinical samples were detected and their concentration values were calculated through the standard curves and subjected to the correlation evaluated against the clinical background values, the performance difference between the two was evaluated, and the results were shown in Figures 1 and 2 (magnetic particle chemiluminescence platform).

In comparison of line width of the traced antigen, the two reagents could meet the market demands (0.02-100mg/L), and the two reagents showed equivalent performance in evaluation of sample correlation.

It could be seen that the detection range of the kit of the present invention could reach 0.02 mg/L to 100 mg/L after the sample treatment solution (citric acid solution with a concentration of 0.1 to 1 M, pH=3 to 4) was added in one step in the reaction process of the kit of the present invention, so that the kit met the requirements of full-range detection of C-reactive protein.

## Claims

1. A kit for full-range detection of C-reactive protein, which comprises:
an M reagent, comprising 0.5~1mg/mL magnetic particles coated with a first antibody, 0.04~0.06% (w/v) surfactant, wherein the surfactant is optionally Tween-20, and 8~12% (w/v) sucrose, its solvent is a phosphate buffer with pH=7.0~8.0; wherein the coating amount of the first antibody is 5~20µg/mg magnetic particles;
an R1 reagent, that is a sample treatment solution, which is a citric acid solution with a concentration of 0.1~1M, pH=3.0~4.0;
an R2 reagent, comprising acridinium ester coated with a secondary antibody, 0.5-1% casein and 0.5-1% bovine serum albumin, its solvent is a phosphate buffer with pH=7.0-8.0, wherein the coating amount of the secondary antibody is 0.3-0.9µg/µg acridinium ester;
a pre-excitation solution and an excitation solution, wherein the pre-excitation solution is 1% (w/v) hydrogen peroxide solution, and the excitation solution is 1 mol/L sodium hydroxide solution;
wherein the first antibody and the second antibody are both monoclonal antibodies that can specifically react with C-reactive protein, and the first antibody and the second antibody are directed to different epitopes;
wherein the first antibody is 10C11 and the second antibody is 14D9-2.

2. A kit for full-range detection of C-reactive protein, which comprises:
a flat-bottomed plate-type chemiluminescence plate coated with a first antibody, which comprises a plate-type luminescence plate, optionally, a 96-well, 384-well or other plate-type luminescence plate, wherein the coating amount of the first antibody is 100~500ng/well, optionally 500ng/well, the coating buffer is a phosphate buffer with pH=7.0~8.0, the blocking solution is 50mM phosphate buffer with pH of 7.2-7.4 comprising 5-8% (w/v) blocking serum or blocking protein, wherein the blocking serum is optionally calf serum, and 0.02% (w/v) sodium azide;
a sample treatment solution, which is a citric acid solution with a concentration of 0. 1 - I M, pH=3 -4;
a labeling enzyme solution, comprising a secondary antibody labeled with horseradish peroxidase or alkaline phosphatase, and having a labeling amount that 1 mg/mL of the secondary antibody is labeled with horseradish peroxidase or alkaline phosphatase in the same proportion;
a color developing solution:
when the labeling enzyme is horseradish peroxidase, the color developing solution comprises a color developing solution A and a color developing solution B, and
the color developing solution A is hydrogen peroxide, optionally, wherein the formula of the color developing solution A: 13.6g of sodium acetate, 1.6g of citric acid, 0.3ml of 30% hydrogen peroxide, formulated with distilled water to 500ml,
the color developing solution B is o-phenylenediamine, optionally, wherein the formula of the color developing solution B: 0.2 g of disodium ethylenediaminetetraacetate, 0.95g of citric acid, 50ml of glycerol, 9.15g of tetramethylbenzidine, formulated with distilled water to 500ml;
when the labeling enzyme is alkaline phosphatase, the color developing solution is a reagent purchased from: Xiamen Boson Biotechnology Co., Ltd. with Art. No.: 180309-01;
wherein the first antibody and the second antibody are both monoclonal antibodies that can specifically react with C-reactive protein, and the first antibody and the second antibody are directed to different epitopes;
wherein the first antibody is 10C11 and the second antibody is 14D9-2.

3. The kit according to claim 1 or 2, wherein the pH of the citric acid solution is adjusted by disodium hydrogen phosphate dodecahydrate, preferably, the pH of the citric acid solution is 3.0-3.5, more preferably, the pH of the citric acid solution is 3.2, 3.3, 3.4 or 3.5.

4. The kit according to any one of claims 1 to 3, wherein the concentration of the citric acid is 0.5 mol/L.

5. The kit according to any one of claims 1 and 3-4 (when dependent on claim 1), wherein:
the method for preparing the M reagent comprises:
the first antibody and the magnetic particles are mixed in 2-morpholineethanesulfonic acid buffer with pH=5.0~6.0, coated at 25-37°C for 1-3h, added with 0.1%~0.5% (w/v) bovine serum albumin phosphate buffer with pH=8.0~9.0 to perform termination for 1~3h,
the coated magnetic particles are separated and dispersed in a phosphate buffer with pH=7.0~8.0, then added with 0.04~0.06% (w/v) surfactant, wherein the surfactant is optionally Tween-20; optionally the surfactant is 0.05% (w/v) Tween-20 and 8~12% (w/v) sucrose, optionally, 10% (w/v) sucrose, to obtain the M reagent;
the method for preparing the R2 reagent comprises:
the second antibody and acridinium ester are mixed in a phosphate buffer with pH=8.0~9.0, coated at 25-37°C for 1~3h, and then added with a Tris buffer comprising 0.1%~0.5% (w/v) bovine serum albumin and having pH=8.0~9.0 to perform termination for 1~3h so as to obtain a stock solution, and
the stock solution is diluted with a phosphate buffer having pH=7.0~8.0 to 1:100-500 to obtain the R2 reagent.

6. The kit according to any one of claims 2 and 3-4 (when depending on claim 2), wherein:
the method for preparing the luminescent plate coating source comprises:
the coated first antibody is diluted with a phosphate buffer having pH=7.0-8.0 as coating buffer to 100-500ng/well (optionally, 500ng/well),
added to the luminescent plate, 100µL per well, incubated at 37°C for 2h or 4°C overnight,
the coating buffer is poured out,
200µL of the blocking solution comprising 5-8% (w/v) calf serum and 0.02% (w/v) sodium azide is used for incubation at 37°C for 2h,
the liquid in the wells is poured out,
the plate is dried and sealed under vacuum with aluminum film, and stored in a dry place at 4°C;
the method for preparing the labeling enzyme solution comprises:
the second antibody and horseradish peroxidase or alkaline phosphatase in ratio of 1:1 are mixed and labeled and dialyzed in a carbonate buffer with pH=9.6, and
the dialysis buffer is replaced every 4 hours and replaced for three times,
the enzyme-labeled secondary antibody is collected to be a stock solution, and
then the stock solution is diluted with an enzyme diluent purchased from Beijing Wantai Biopharmaceutical Co., Ltd. with Cat. No. ED-11 to 1:500 to obtain the labeling enzyme solution.

7. A method for full-range detection of C-reactive protein, which is performed by using the kit according to any one of claims 1 and 3-4 (when depending on claim 1), comprising:
(1) 20µL of a sample is taken and added to 100µL of the R1 reagent to treat the sample, wherein the sample is serum;
(2) 50µL of the M reagent is then added and incubated together for 15min;
(3) after step (2), washing is performed with a phosphate buffer comprising 0.05~0.08% (w/v) Tween-20, then 50 µL of the R2 reagent is added and incubated for 10 minutes;
(4) after step (3), washing is performed with a phosphate buffer comprising 0.05~0.08% (w/v) Tween-20, and 100 µL of the pre-excitation solution is added to perform pre-excitation;
(5) the pre-excitation solution is removed, 100 µL of the excitation solution is then added to perform excitation and detection.

## Patentansprüche

1. Kit für den Vollbereichsnachweis von C-reaktivem Protein, das Folgendes umfasst:
ein Reagenz M, umfassend 0,5 bis 1 mg/ml magnetische Partikel, die mit einem ersten Antikörper beschichtet sind, 0,04 bis 0,06 % (w/v) Tensid, wobei das Tensid gegebenenfalls Tween-20 ist, und 8 bis 12 % (w/v) Saccharose, wobei das Lösungsmittel ein Phosphatpuffer mit pH=7,0 bis 8,0 ist; wobei die Beschichtungsmenge des ersten Antikörpers 5 bis 20 µg/mg magnetische Partikel beträgt;
ein Reagenz R1, bei dem es sich um eine Probenbehandlungslösung handelt, die eine Zitronensäurelösung mit einer Konzentration von 0,1 bis 1 M, pH=3,0 bis 4,0, ist;
ein Reagenz R2, umfassend Acridiniumester, beschichtet mit einem sekundären Antikörper, 0,5 bis 1 % Casein und 0,5 bis 1 % Rinderserumalbumin, dessen Lösungsmittel ein Phosphatpuffer mit pH=7,0 bis 8,0 ist, wobei die Beschichtungsmenge des sekundären Antikörpers 0,3 bis 0,9 µg/µg Acridiniumester beträgt;
eine Voranregungslösung und eine Anregungslösung, wobei die Voranregungslösung eine 1 %ige (w/v) Wasserstoffperoxidlösung und die Anregungslösung eine Natriumhydroxidlösung mit einer Konzentration von 1 mol/l ist;
wobei der erste Antikörper und der zweite Antikörper beide monoklonale Antikörper sind, die spezifisch mit C-reaktivem Protein reagieren können, und der erste Antikörper und der zweite Antikörper gegen unterschiedliche Epitope gerichtet sind;
wobei der erste Antikörper 10C11 und der zweite Antikörper 14D9-2 ist.

2. Kit für den Vollbereichsnachweis von C-reaktivem Protein, das Folgendes umfasst:
eine plattenförmige Chemilumineszenzplatte mit flachem Boden, die mit einem ersten Antikörper beschichtet ist, die eine plattenförmige Lumineszenzplatte, gegebenenfalls eine 96-Well-, 384-Well- oder eine andere plattenförmige Lumineszenzplatte umfasst, wobei die Beschichtungsmenge des ersten Antikörpers 100 bis 500 ng/Well, gegebenenfalls 500 ng/Well, beträgt, der Beschichtungspuffer ein Phosphatpuffer mit pH=7,0 bis 8,0 ist und die Blockierlösung ein 50 mM Phosphatpuffer mit einem pH-Wert von 7,2 bis 7,4 ist, umfassend 5 bis 8 % (w/v) Blockierserum oder Blockierprotein, wobei das Blockierserum optional Kälberserum ist, und 0,02 % (w/v) Natriumazid;
eine Probenbehandlungslösung, bei dem es sich um eine Zitronensäurelösung mit einer Konzentration von 0,1 bis 1 M, pH=3 bis 4, handelt;
eine Markierungsenzymlösung, die einen mit Meerrettichperoxidase oder alkalischer Phosphatase markierten sekundären Antikörper umfasst und eine Markierungsmenge aufweist, bei der 1 mg/ml des sekundären Antikörpers mit Meerrettichperoxidase oder alkalischer Phosphatase im gleichen Verhältnis markiert wird;
eine Farbentwicklungslösung:
wenn das Markierungsenzym Meerrettichperoxidase ist, umfasst die Farbentwicklungslösung eine Farbentwicklungslösung A und eine Farbentwicklungslösung B, und
die Farbentwicklungslösung A ist optional Wasserstoffperoxid, wobei die Formel der Farbentwicklungslösung A wie folgt ist: 13,6 g Natriumacetat, 1,6 g Zitronensäure, 0,3 ml 30 %iges Wasserstoffperoxid, mit destilliertem Wasser auf 500 ml aufgefüllt,
die Farbentwicklungslösung B ist optional o-Phenylendiamin, wobei die Formel der Farbentwicklungslösung B wie folgt ist: 0,2 g Dinatriumethylendiamintetraacetat, 0,95 g Zitronensäure, 50 ml Glycerin, 9,15 g Tetramethylbenzidin, mit destilliertem Wasser auf 500 ml aufgefüllt;
wenn es sich bei dem markierenden Enzym um alkalische Phosphatase handelt, ist die Farbentwicklungslösung ein Reagenz, das von folgendem Hersteller bezogen wird: Xiamen Boson Biotechnology Co., Ltd. mit der Art.-Nr.: 180309-01;
wobei der erste Antikörper und der zweite Antikörper beide monoklonale Antikörper sind, die spezifisch mit C-reaktivem Protein reagieren können, und der erste Antikörper und der zweite Antikörper gegen unterschiedliche Epitope gerichtet sind;
wobei der erste Antikörper 10C11 und der zweite Antikörper 14D9-2 ist.

3. Kit nach Anspruch 1 oder 2, wobei der pH-Wert der Zitronensäurelösung durch Dinatriumhydrogenphosphat-Dodecahydrat eingestellt wird, wobei der pH-Wert der Zitronensäurelösung vorzugsweise 3,0 bis 3,5, noch bevorzugter 3,2, 3,3, 3,4 oder 3,5, beträgt.

4. Kit nach einem der Ansprüche 1 bis 3, wobei die Konzentration der Zitronenlösung 0,5 mol/l beträgt.

5. Kit nach einem der Ansprüche 1 und 3 bis 4 (wenn abhängig von Anspruch 1), wobei:
das Verfahren zum Herstellen des Reagenz M Folgendes umfasst:
der erste Antikörper und die magnetischen Partikel werden in 2-Morpholinethansulfonsäure-Puffer mit pH=5,0 bis 6,0 gemischt, bei 25 bis 37 °C für 1 bis 3 h beschichtet und mit 0,1 % bis 0,5 % (w/v) Rinderserumalbumin-Phosphatpuffer mit pH=8,0 bis 9,0 versetzt, um die Terminierung für 1 bis 3 h durchzuführen,
die beschichteten magnetischen Teilchen werden abgetrennt und in einem Phosphatpuffer mit einem pH-Wert von 7,0 bis 8,0 dispergiert und dann mit 0,04 bis 0,06 % (w/v) Tensid versetzt, wobei das Tensid optional Tween-20 ist; wobei das Tensid optional 0,05 % (w/v) Tween-20 und 8 bis 12 % (w/v)
Saccharose, optional 10 % (w/v) Saccharose, ist, um das Reagenz M zu erhalten;
das Verfahren zum Herstellen des Reagenz R2 Folgendes umfasst:
der zweite Antikörper und der Acridiniumester werden in einem Phosphatpuffer mit einem pH-Wert von 8,0 bis 9,0 gemischt, 1 bis 3 Stunden lang bei 25 bis 37 °C beschichtet und dann mit einem Tris-Puffer, der 0,1 bis 0,5 % (w/v) Rinderserumalbumin enthält und einen pH-Wert von 8,0 bis 9,0 aufweist, versetzt, um 1 bis 3 Stunden lang eine Terminierung durchzuführen, um eine Stammlösung zu erhalten, und
die Stammlösung wird mit einem Phosphatpuffer mit einem pH-Wert von 7,0 bis 8,0 im Verhältnis 1:100 bis 500 verdünnt, um das Reagenz R2 zu erhalten.

6. Kit nach einem der Ansprüche 2 und 3 bis 4 (wenn abhängig von Anspruch 2), wobei:
das Verfahren zum Herstellen der lumineszierenden Plattenbeschichtungsquelle Folgendes umfasst:
der beschichtete erste Antikörper wird mit einem Phosphatpuffer mit einem pH-Wert von 7,0 bis 8,0 als Beschichtungspuffer auf 100-500 ng/Well (optional 500 ng/Well) verdünnt,
in die lumineszierende Platte gegeben, 100 µl pro Well, bei 37 °C für 2 h oder 4 °C über Nacht inkubiert,
der Beschichtungspuffer wird ausgegossen,
200 µl der Blockierlösung, die 5 bis 8 % (w/v) Kälberserum und 0,02 % (w/v) Natriumazid enthält, werden für eine 2-stündige Inkubation bei 37 °C verwendet,
die Flüssigkeit in den Wells wird ausgegossen,
die Platte wird getrocknet und unter Vakuum mit Aluminiumfolie versiegelt und an einem trockenen Ort bei 4 °C gelagert;
das Verfahren zum Herstellen der Lösung des Markierungsenzyms Folgendes umfasst:
der zweite Antikörper und Meerrettichperoxidase oder alkalische Phosphatase werden im Verhältnis 1:1 gemischt und markiert und in einem Carbonatpuffer mit pH=9,6 dialysiert, und
der Dialysepuffer wird alle 4 Stunden ersetzt und dreimal ausgetauscht,
der enzym markierte sekundäre Antikörper wird als Stammlösung aufgefangen, und
anschließend wird die Stammlösung mit einem Enzymverdünnungsmittel, bezogen vom Hersteller Beijing Wantai Biopharmaceutical Co., Ltd., mit der Art.- Nr. ED-11, auf 1:500 verdünnt, um die Markierungsenzymlösung zu erhalten.

7. Verfahren zum Vollbereichsnachweis von C-reaktivem Protein, das unter Verwendung des Kits nach einem der Ansprüche 1 und 3 bis 4 (wenn abhängig von Anspruch 1) durchgeführt wird und Folgendes umfasst:
(1) 20 µl einer Probe werden entnommen und zu 100 µl des Reagenzes R1 hinzugefügt, um die Probe zu behandeln, wobei die Probe Serum ist;
(2) anschließend werden 50 µl des Reagenzes M hinzugefügt und 15 Minuten lang inkubiert;
(3) nach Schritt (2) wird mit einem Phosphatpuffer gewaschen, der 0,05 bis 0,08 % (w/v) Tween-20 enthält, dann werden 50 µl des Reagenzes R2 hinzugefügt und 10 Minuten lang inkubiert;
(4) nach Schritt (3) wird mit einem Phosphatpuffer gewaschen, der 0,05 bis 0,08 % (w/v) Tween-20 enthält, und 100 µl der Voranregungslösung werden hinzugefügt, um eine Voranregung durchzuführen;
(5) die Voranregungslösung wird entfernt, dann werden 100 µl der Anregungslösung hinzugefügt, um die Anregung und Detektion durchzuführen.

## Revendications

1. Kit pour la détection complète de protéine C réactive, qui comprend :
un réactif M, comprenant 0,5 à 1 mg/mL de particules magnétiques revêtues d'un premier anticorps, 0,04 à 0,06 % (p/v) de tensioactif, le tensioactif étant éventuellement du Tween-20, et 8 à 12 % (p/v) de saccharose, son solvant étant un tampon phosphate de pH = 7,0 à 8,0 ; la quantité de revêtement du premier anticorps étant de 5 à 20 µg/mg de particules magnétiques ;
un réactif R1, c'est-à-dire une solution de traitement d'échantillon, qui est une solution d'acide citrique avec une concentration de 0,1 à 1M, de pH = 3,0 à 4,0 ;
un réactif R2, comprenant un ester d'acridinium revêtu d'un anticorps secondaire, 0,5 à 1 % de caséine et 0,5 à 1 % d'albumine de sérum bovin, son solvant est un tampon phosphate de pH = 7,0 à 8,0, la quantité de revêtement de l'anticorps secondaire étant de 0,3 à 0,9 µg/µg d'ester d'acridinium ;
une solution de pré-excitation et une solution d'excitation, la solution de pré-excitation étant une solution de peroxyde d'hydrogène à 1 % (p/v) et la solution d'excitation étant une solution d'hydroxyde de sodium à 1 mol/L ;
le premier anticorps et le second anticorps étant tous deux des anticorps monoclonaux qui peuvent réagir spécifiquement avec la protéine C réactive, et le premier anticorps et le second anticorps étant dirigés vers des épitopes différents ;
le premier anticorps étant 10C11 et le second anticorps étant 14D9-2.

2. Kit pour la détection complète de protéine C réactive, qui comprend :
une plaque de chimiluminescence de type plaque à fond plat revêtue d'un premier anticorps, qui comprend une plaque de luminescence de type plaque, éventuellement une plaque de luminescence de type plaque à 96 puits, 384 puits ou autre, la quantité de revêtement du premier anticorps étant de 100 à 500 ng/puits, éventuellement 500 ng/puits, le tampon de revêtement étant un tampon phosphate de pH = 7,0 à 8,0, la solution de blocage étant un tampon phosphate à 50mM de pH de 7,2 à 7,4 comprenant 5 à 8 % (p/v) de sérum de blocage ou de protéine de blocage, le sérum de blocage étant éventuellement du sérum de veau, et 0,02 % (p/v) d'azoture de sodium ;
une solution de traitement d'échantillon, qui est une solution d'acide citrique avec une concentration de 0,1 à 1M, pH = 3 à 4 ;
une solution d'enzyme de marquage, comprenant un anticorps secondaire marqué avec de la peroxydase de raifort ou de la phosphatase alcaline, et ayant une quantité de marquage telle que 1 mg/mL de l'anticorps secondaire est marqué avec de la peroxydase de raifort ou de la phosphatase alcaline dans la même proportion ;
une solution de développement de couleur :
lorsque l'enzyme de marquage est la peroxydase de raifort, la solution de développement de couleur comprend une solution de développement de couleur A et une solution de développement de couleur B, et
la solution de développement de couleur A est le peroxyde d'hydrogène, éventuellement, la formule de la solution de développement de couleur A : 13,6 g d'acétate de sodium, 1,6 g d'acide citrique, 0,3 mL de peroxyde d'hydrogène à 30 %, formulée avec de l'eau distillée à 500 mL,
la solution de développement de couleur B est l'o-phénylènediamine, éventuellement, la formule de la solution de développement de couleur B : 0,2 g d'éthylènediaminetétraacétate de disodium, 0,95 g d'acide citrique, 50 mL de glycérol, 9,15 g de tétraméthylbenzidine, formulée avec de l'eau distillée à 500 mL ;
lorsque l'enzyme de marquage est la phosphatase alcaline, la solution de développement de couleur est un réactif acheté auprès de : Xiamen Boson Biotechnology Co., Ltd. avec le n° d'article : 180309-01 ;
le premier anticorps et le second anticorps étant tous deux des anticorps monoclonaux qui peuvent réagir spécifiquement avec la protéine C réactive, et le premier anticorps et le second anticorps étant dirigés vers des épitopes différents ;
le premier anticorps étant 10C11 et le second anticorps étant 14D9-2.

3. Kit selon la revendication 1 ou 2, dans lequel le pH de la solution d'acide citrique est ajusté par de l'hydrogénophosphate de disodium dodécahydraté, de préférence, le pH de la solution d'acide citrique est de 3,0 à 3,5, plus préférentiellement, le pH de la solution d'acide citrique est de 3,2, 3,3, 3,4 ou 3,5.

4. Kit selon l'une quelconque des revendications 1 à 3, dans lequel la concentration de l'acide citrique est de 0,5 mol/L.

5. Kit selon l'une quelconque des revendications 1 et 3 à 4 (lorsqu'elles dépendent de la revendication 1), dans lequel :
le procédé de préparation du réactif M comprend :
le mélange du premier anticorps et des particules magnétiques dans un tampon acide 2-morpholineéthanesulfonique de pH = 5,0 à 6,0, leur revêtement à 25 à 37°C pendant 1 à 3 h, l'ajout de 0,1 % à 0,5 % (p/v) d'albumine de sérum bovin dans un tampon phosphate de pH = 8,0 à 9,0 pour réaliser une terminaison pendant 1 à 3 h,
la séparation des particules magnétiques revêtues et leur dispersion dans un tampon phosphate de pH = 7,0 à 8,0, puis l'ajout de 0,04 à 0,06 % (p/v) de tensioactif, le tensioactif étant éventuellement du Tween-20 ; le tensioactif étant éventuellement du Tween-20 à 0,05 % (p/v) et du saccharose à 8 à 12 % (p/v),
éventuellement du saccharose à 10 % (p/v), pour obtenir le réactif M ;
le procédé de préparation du réactif R2 comprend :
le mélange du second anticorps et de l'ester d'acridinium dans un tampon phosphate de pH = 8,0 à 9,0, leur revêtement à 25 à 37 °C pendant 1 à 3 h, puis l'ajout d'un tampon Tris comprenant 0,1 % à 0,5 % (p/v) d'albumine de sérum bovin et ayant un pH = 8,0 à 9,0 pour réaliser une terminaison pendant 1 à 3 h afin d'obtenir une solution mère, et
la dilution de la solution mère avec un tampon phosphate ayant un pH = 7,0 à 8,0 à 1:100 à 500 pour obtenir le réactif R2.

6. Kit selon l'une quelconque des revendications 2 et 3 à 4 (lorsqu'elles dépendent de la revendication 2), dans lequel :
le procédé de préparation de la source de revêtement de plaque luminescente comprend :
la dilution du premier anticorps revêtu avec un tampon phosphate ayant un pH = 7,0 à 8,0 en tant que tampon de revêtement à 100 à 500 ng/puits (éventuellement, 500 ng/puits),
son ajout à la plaque luminescente, 100 µL par puits, son incubation à 37 °C pendant 2 h ou 4 °C pendant une nuit,
la vidange du tampon de revêtement,
l'utilisation de 200 µL de la solution de blocage comprenant 5 à 8 % (w/v) de sérum de veau et 0,02 % (w/v) d'azoture de sodium pour une incubation à 37 °C pendant 2 h,
la vidange du liquide contenu dans les puits,
le séchage de la plaque et son scellage sous vide avec un film d'aluminium, et son stockage dans un endroit sec à 4 °C ;
le procédé de préparation de la solution d'enzyme de marquage comprend :
la mélange du second anticorps et de la peroxydase de raifort ou de la phosphatase alcaline dans un rapport de 1:1, leur marquage et leur dialyse dans un tampon carbonate de pH = 9,6, et
le remplacement du tampon de dialyse toutes les 4 heures trois fois,
le recueil de l'anticorps secondaire marqué par enzyme pour en faire une solution mère, et
la dilution de la solution mère avec un diluant enzymatique acheté auprès de Beijing Wantai Biopharmaceutical Co., Ltd ayant le n° de catégorie ED-11 à 1:500 pour obtenir la solution d'enzyme de marquage.

7. Procédé pour la détection complète de la protéine C-réactive, qui est réalisé à l'aide du kit selon l'une quelconque des revendications 1 et 3 à 4 (lorsqu'elles dépendent de la revendication 1), comprenant :
(1) le prélèvement de 20 µL d'un échantillon l'ajout à 100µL du réactif R1 pour traiter l'échantillon, l'échantillon étant du sérum ;
(2) puis l'ajout de 50 µL du réactif M et leur incubation ensemble pendant 15 min ;
(3) après l'étape (2), la réalisation d'un lavage avec un tampon phosphate comprenant 0,05 à 0,08 % (w/v) de Tween-20, puis l'ajout de 50 µL du réactif R2 et leur incubation pendant 10 minutes ;
(4) après l'étape (3), la réalisation d'un lavage avec un tampon phosphate comprenant 0,05 à 0,08 % (w/v) de Tween-20, et l'ajout de 100 µL de la solution de pré-excitation pour réaliser une pré-excitation ;
(5) l'élimination de la solution de pré-excitation, puis l'ajout de 100 µL de la solution d'excitation pour réaliser une excitation et une détection.
